# EUROPEAN PATENT SPECIFICATION

(11) **EP 3 280 403 B1**
(45) Date of publication and mention of the grant of the patent: **29.07.2020**
(21) Application number: 16777243.3
(22) Date of filing: 07.04.2016
(51) Int. Cl.: A61K 31/05, A61K 31/085, A61P 31/12

(54) **THERAPEUTIC APPLICATIONS OF PRENYLATED STILBENOIDS AGAINST ROTAVIRUS INFECTIONS**
THERAPEUTISCHE ANWENDUNGEN VON PRENYLIERTEN STILBENOIDEN GEGEN ROTAVIRUSINFEKTIONEN
APPLICATIONS THÉRAPEUTIQUES DE STILBÉNOÏDES PRÉNYLÉS CONTRE LES INFECTIONS À ROTAVIRUS

(30) Priority: 08.04.2015 US 201562144390 P; 06.04.2016 US 201615091758
(43) Date of publication of application: 14.02.2018
(73) Proprietor: Arkansas State University, Jonesboro, AR 72467 (US)
(72) Inventor: MEDINA-BOLIVAR, Luis, Fabricio, Memphis, Tennessee 38112 (US); PARR, Rebecca, D., College Station, Texas 77845 (US)
(74) Representative: McNab, Donald C.
(86) International application number: PCT/US2016/026346
(87) International publication number: WO 2016/164525

(56) References cited:
- US-A1- 2012 165 280
- US-A1- 2012 165 281
- US-A1- 2015 025 041
- US-B2- 7 384 920
- BALL JUDITH M ET AL: "Investigation of Stilbenoids as Potential Therapeutic Agents for Rotavirus Gastroenteritis.", ADVANCES IN VIROLOGY, vol. 2015, no. 293524, 2015, pages 1-10, XP002784987, ISSN: 1687-8639, DOI: 10.1155/2015/293524
- KWON ET AL.: 'In vitro anti-rotavirus activity of polyphenol compounds isolated from the roots of Glycyrrhiza uralensis' BIOORGANIC & MEDICINAL CHEMISTRY vol. 18, no. 21, 2010, pages 7668 - 7674, XP027415421

## Description

The present invention is defined by the appended claims.

### FIELD OF THE INVENTION

The present invention relates generally to the treatment of virus infections. More specifically, the present invention relates to compositions and methods to treat rotavirus infections.

### BACKGROUND OF THE INVENTION

Of the various enteric pathogenic viruses causing severe diarrhea in children, rotavirus is the most common causing an average of 611,000 deaths per year. Virtually all children are infected by rotavirus by age 5. The virus is believed to be highly contagious and has been described as a "democratic" virus since the infection affects no particular socioeconomic or geographic group disproportionately. While the majority of children having access to adequate supportive and palliative medical care survive infection with no significant long-term consequences, the number of deaths associated with severe diarrhea, vomiting, dehydration and shock is unacceptable and requires preventative intervention if possible. What is needed in the art are methods and therapeutics for the treatment of rotavirus infections.

### SUMMARY OF THE INVENTION

The present invention relates generally to the treatment of virus infections. More specifically, the present invention relates to compositions and methods to treat rotavirus infections.

In one embodiment, the present invention contemplates a prenylated stilbenoid for use in a method for inhibiting a rotavirus infection in a subject, comprising administering to said subject exhibiting at least one symptom of a rotavirus infection a pharmaceutically effective amount of at least one prenylated stilbenoid, under conditions such that said at least one symptom of a rotavirus infection is reduced. In one embodiment, said at least one prenylated stilbenoid comprises a combination of prenylated stilbenoids. In one embodiment, said at least one prenylated stilbenoid is a purified stilbenoid. In one embodiment, said at least one prenylated stilbenoid comprises an extract of hairy root cultures of a peanut plant. In one embodiment, said at least one prenylated stilbenoid comprises an enriched fraction of a stilbenoid extract. In one embodiment, said prenylated stilbenoid is selected from the group consisting of arachidin-1 and arachidin-3. In one embodiment, said prenylated stilbenoid is purified from a biological system. In one embodiment, said biological system comprises a natural and/or a transgenic biological system producing the prenylated compounds. In one embodiment, said extract enriched in prenylated stilbenoids is obtained from a biological system. In one embodiment, said biological system is a hairy root culture. In one embodiment, said prenylated stilbenoid is produced synthetically. In one embodiment, said prenylated stilbenoid modulates at least one cannabinoid receptor. In one embodiment, said modulation of at least one cannabinoid receptor affects virus replication. In one embodiment, said prenylated stilbenoid inhibits autophagy in virus-infected cells. In one embodiment, said inhibition of autophagy affects virus replication. In one embodiment, said administering is intravenous. In one embodiment, said administering is intraperitoneal. In one embodiment, said administering is oral. In one embodiment, said administering comprises the use of a delivery system. In one embodiment, said delivery system includes, but is not limited to, an emulsion or a nanoparticle.

In one embodiment, the present invention contemplates a prenylated stilbenoid for use in a method for inhibiting a virus infection in a subject, comprising administering to said subject exhibiting at least one symptom of a virus infection a pharmaceutically effective amount of at least one purified prenylated stilbenoid, under conditions such that said at least one symptom of a virus infection is reduced. In one embodiment, said at least one prenylated stilbenoid comprises a combination of prenylated stilbenoids. In one embodiment, said at least one prenylated stilbenoid is a purified stilbenoid. In one embodiment, said at least one prenylated stilbenoid comprises an extract of hairy root cultures of the peanut plant. In one embodiment, said at least one prenylated stilbenoid comprises an enriched fraction of a stilbenoid extract. In one embodiment, said prenylated stilbenoid is selected from the group consisting of arachidin-1 and arachidin-3. In one embodiment, said prenylated stilbenoid is purified from a biological system. In one embodiment, said extract enriched in prenylated stilbenoids is obtained from a biological system. In one embodiment, said biological system is a hairy root culture. In one embodiment, said prenylated stilbenoid is produced synthetically. In one embodiment, said prenylated stilbenoid modulates at least one cannabinoid receptor. In one embodiment, said modulation of at least one cannabinoid receptor reduces virus replication. In one embodiment, said prenylated stilbenoid inhibits autophagy in virus infected cells. In one embodiment, said inhibition of autophagy reduces virus replication. In one embodiment, said virus is a rotavirus. In one embodiment, said administering is intravenous. In one embodiment, said administering is intraperitoneal. In one embodiment, said administering is oral.

In one embodiment, the present invention contemplates a prenylated stilbenoid for use in a method for inhibiting a viral infection in a subject, comprising administering to said subject exhibiting at least one symptom of a viral infection a pharmaceutically effective amount of at least one purified prenylated stilbenoid, under conditions such that said at least one symptom of a viral infection is reduced. In one embodiment, said at least one prenylated stilbenoid comprises a combination of prenylated stilbenoids. In one embodiment, said at least one prenylated stilbenoid is a purified stilbenoid. In one embodiment, said at least one prenylated stilbenoid comprises an extract of hairy root cultures of the peanut plant. In one embodiment, said at least one prenylated stilbenoid comprises an enriched fraction of a stilbenoid extract. In one embodiment, said prenylated stilbenoid is selected from the group consisting of arachidin-1 and arachidin-3. In one embodiment, said viral infection comprises a infection by a virus selected of from the group consisting of picornaviruses (poliovirus, rhinovirus, hepatitis A), coronaviruses, flaviviruses (hepatitis C, yellow fever, dengue fever, dengue virus and west nile virus), and zika virus. While not limiting the current invention, it is belived that the antiviral (rotavirus) mechanism is by inhibition of autophagy. As mentioned below, many viruses exploit autophagy to promote viral replication. This includes important RNA viruses such as picornaviruses (poliovirus, rhinovirus, hepatitis A), coronaviruses, flaviviruses (hepatitis C, yellow fever, dengue fever, dengue virus and west nile virus). There is also information about zika virus and autophagy. It is believed that the prenylated stilbenods may have a similar effect because these other viruses use a similar mechanism to promote their replication.

The described features, structures, or characteristics of the invention may be combined in any suitable manner in one or more embodiments. In the following description, numerous specific details are recited to provide a thorough understanding of embodiments of the invention. One skilled in the relevant art may recognize, however, that the invention may be practiced without one or more of the specific details, or with other methods, components, materials, and so forth. In other instances, well-known structures, materials, or operations are not shown or described in detail to avoid obscuring embodiments of the invention.

Other objects, advantages, and novel features, and further scope of applicability of the present invention will be set forth in part in the detailed description to follow, taken in conjunction with the accompanying drawings, and in part will become apparent to those skilled in the art upon examination of the following, or may be learned by practice of the invention. The objects and advantages of the invention may be realized and attained by means of the instrumentalities and combinations particularly pointed out in the appended claims.

### DEFINITIONS

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

As used herein, the term "prenylated stilbenoid" refers to a monomeric or oligomomeric hydroxylated derivative of a stilbene containing one or multiple prenylated side chains. The prenylated side chain includes but it is not limited to a single isopentenyl moiety (3-methyl-1-butenyl) as in arachidin-1 and arachidin-3, a single prenylated moiety (3-methyl-but-2-en-1-yl) as in arachidin-2, a single isopentadienyl moiety as in isopentadienyl resveratrol (IPD) or derivatives and combinations of these moieties. Prenylated stilbenoids can include single or multiple prenylated side chains.

As used herein, the term "patient" or "subject" or "host" refers to a living animals with a rotavirus infection or at risk of exposure to rotavirus infection. In preferred embodiments, said animals comprise mammalian organism, such as a human, monkey, cow, sheep, goat, dog, cat, mouse, rat, guinea pig, or transgenic species thereof. In certain embodiments, the patient or subject is a primate. Non-limiting examples of human subjects are adults, juveniles, infants and fetuses.

As used herein, the terms "reduce," "inhibit," "diminish," "suppress," "decrease," "prevent" and grammatical equivalents (including "lower," "smaller," etc.) when in reference to the expression of any symptom in an untreated subject relative to a treated subject, mean that the quantity and/or magnitude of the symptoms in the treated subject is lower than in the untreated subject by any amount that is recognized as clinically relevant by any medically trained personnel. In one embodiment, the quantity and/or magnitude of the symptoms in the treated subject is at least 10% lower than, at least 25% lower than, at least 50% lower than, at least 75% lower than, and/or at least 90% lower than the quantity and/or magnitude of the symptoms in the untreated subject.

As used herein, the term "prevention" or "preventing" is used throughout the specification to include: (1) inhibiting the onset of a disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

As used herein, the terms "treat" and "treating" are not limited to the case where the subject (e.g. patient) is cured and the disease is eradicated. Rather, the present invention also contemplates treatment that merely reduces symptoms, improves (to some degree) and/or delays disease progression. It is not intended that the present invention be limited to instances wherein a disease or affliction is cured. It is sufficient that symptoms are reduced.

As used herein, the terms "medication" or "therapeutic agent" refer to a compound that treats or prevents or alleviates the symptoms of disease or condition. For example, a compound may include, but is not limited to, a drug or pharmaceutical composition. Medication is considered to be delivered or present in therapeutically effective amounts or pharmaceutically effective amounts.

The present invention contemplates the above-described compositions in "therapeutically effective amounts" or "pharmaceutically effective amounts", which means that amount which, when administered to a subject or patient for treating a disease, is sufficient to effect such treatment for the disease or to ameliorate one or more symptoms of a disease or condition (e.g. ameliorate pain).

As used herein, the term "oral administration" includes oral, buccal, enteral and intra-gastric administration.

As used herein, the term "parenteral administration" includes but is not limited to topical (including administration to any dermal, epidermal or mucosal surface), subcutaneous, intravenous, intraperitoneal, and intramuscular administration.

As used herein, the term "pharmaceutically acceptable carrier" is intended to refer to a carrier including but not limited to an excipient, diluent or auxiliary, or combination thereof, that can be administered to a subject as a component of a composition described herein that does not reduce the activity of the composition and is not toxic when administered in doses sufficient to deliver an effective amount of a compound or composition useful herein. The formulations can be administered orally, nasally or parenterally (including topically, intramuscularly, intraperitoneally, subcutaneously and intravenously).

### DESCRIPTION OF THE FIGURES

The accompanying figures, which are incorporated into and form a part of the specification, illustrate several embodiments of the present invention and, together with the description, serve to explain the principles of the invention. The figures are only for the purpose of illustrating a preferred embodiment of the invention and are not to be construed as limiting the invention.
Figure 1 shows several embodiments of stilbenoid chemical structures. All compounds are shown in trans isomers. A. Resveratrol (t-Res). B. Piceatannol (t-Pa). C. Arachidin-3(t-A3). D. Arachidin-1 (t-A1).
Figure 2A-C shows an exemplary HPLC analysis of stilbenoids. A.HPLC chromatogram of ethyl acetate extract of the medium of hairy root culture of peanut treated with methyl-β-cyclodextrinfor 72 h. Compounds: (1) arachidin-1 and (2) arachidin-3. B. HPLC chromatogram of (1) arachidin-1 purified by HPCCC. C. HPLC chromatogram of (2) arachidin-3 purified by HPCCC.
Figure 3A-D shows exemplary data of a quantification of progeny RV via focus forming units/ml (FFU/ml). HT29.8 cells were infected with RV, with RV with 0.02% DMSO, or 10µM/20µM of A. Resveratrol (t-Res). B. Piceatannol(t-Pa). C. Arachidin-1 (t-A1). D. Arachidin-3(t-A3).
Figure 4A-D shows exemplary data of a quantification of progeny RV in plaque forming units/ml. HT29.8 cells were infected with RV, with RV with 0.02% DMSO, or 20µM of A. Resveratrol (t-Res).B. Piceatannol(t-Pa). C. Arachidin-1 (t-A1).D. Arachidin-3(t-A3). * Statistically significant p = 0.02. ** Statistically significant p = 0.04.

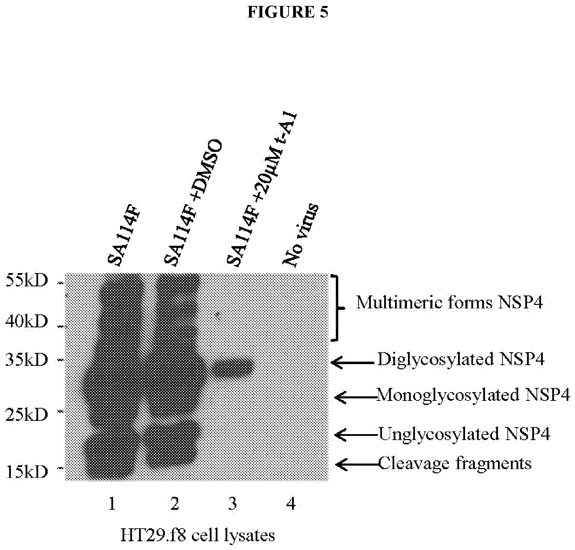

Figure 5 shows exemplary data of a western blot analyses of HT29.f8 cell lysates from RV-infected (lanes 1, 2, 3) and non-infected (lane 4) cells, treated with DMSO (lane 2) or t-A1 in DMSO (lane 3). Cell lysates were separated on SDS PAGE gels and electroblottedonto nitrocellulose. Blots were probed with rabbit anti-NSP4 (150-175). RV-infected cells without and with DMSO, lanes 1 and 2 respectively, show cleavage fragments, unglycosylated, mono, diglycosylatedand multimericforms of NSP4. RV-infected cells treated with 20µM t-A1 with DMSO, lane3, only shows the diglycosylatedform of NSP4. Uninfected and untreated cells in lane 4 show no NSP4 banding patterns.
Figure 6 shows a transmission electron micrograph shows rotavirus (Wa) infected HT29.f8 with 20 mM arachidin-3 at 18 hours post infection. Mitochondria (M), rotavirus particles (RV), and a nucleolus (N) can be seen. Final Magnification = 8,300X.
Figure 7 shows a transmission electron micrograph shows rotavirus (Wa) infected HT29.f8 cells 18 hours post infection. Mitochondria (M) and autophagosomes (AP) can be seen. Final Magnification = 7,800X
Figure 8 shows a transmission electron micrograph shows rotavirus (Wa) infected HT29.f8 cells with 20 mM arachidin-3 at 18 hours post infection. Mitochondria (M) and vacuoles (V) can be seen. A Viroplasm (VP) can be seen where rotavirus (RV) particles are produced. Final Magnification = 10,000X
Figure 9 shows a transmission electron micrograph shows rotavirus (Wa) infected HT29.f8 cells 18 hours post infection. Autophagosomes (AP) and vacuoles (V) can be seen. Final Magnification = 7,600X
Figure 10A&B shows cell lysates (Figure 10A -uninfected HT29.f8; Figure 10B-uninfected MA104); C-RV-infected (Wa) MA104/HT29.f8 cell lysates were added to nitrocellulose membranes, probed with a 1:1000 dilution of rabbit anti-CNR1/2 antibodies from Antibodies Online (Atlanta, GA) and reactive bands were visualized by the addition and excitation of goat anti-rabbit antibodies Alexa Fluor®546 (Life Technologies) using the Typhoon 8600 laser scanner.
Figure 11 shows the fold change in caspase genes expression. Total RNA was extracted at 8 hours post RV infection of HT29.f8 cells with/without arachidin-3 and controls of no virus and arachidin-3 only. Using qRT-PCR, the cDNA was amplified using gene-specific primers to detect caspases 3, 7, 8, 9, & 10 transcripts and normalized with human GAPDH and b-actin. The experiment were performed in duplicate. The green boxes show the results of the caspases that are effected by arachidin-3. The ΔCt values obtained from the qRT-PCR were used to determine the relative fold changes in gene expression.
   NV- no virus + 0.0002% DMSO
   RV- human rotavirus (Wa strain) only + 0.0002% DMSO
   RV+arachidin-3- human rotavirus (Wa strain) with 20 mM arachidin-3
   t-arachidin-3- 20 mM arachidin-3 only

### DESCRIPTON OF THE INVENTION

### 1. CONVENTIONAL UTILITY OF PRENYLATED STILBENOIDS

Condori, J. et al. (2010) Induced Biosynthesis of Resveratrol and the Prenylated Stilbenoids Arachidin-1 and Arachidin-3 in Hairy Root Cultures of Peanut: Effects of Culture Medium and Growth Stage, Plant Physiol. Biochem. 48(5), 310-318 [1]. This reference describes that both stilbenoids arachidin-1 and arachidin-3 are known to be produced in hairy root cultures of the peanut plant and their biosynthesis may be induced. The reference also indicates the usefulness of such stilbenoids because of their antioxidant properties; stilbenoids may provide peanuts and other plants supporting this metabolic pathway, protection against oxidative stress and other environmental challenges including ozone and UV light exposure. While describing both arachidin-1 and trans-arachidin-3, this reference does not describe treatment or prevention of rotavirus infections.

Huang, C.-P. et al. (2010) Arachidin-1, a Peanut Stilbenoid, Induces Programmed Cell Death in Human Leukemia Hl-60 Cells, J. Agric. Food Chem. 58(23), 12123-12129 [2]. This reference describes the use of arachidin-1 in inducing programmed cell death in human leukemia HL-60 cells. The reference suggests the use of other such stilbenoids for anticancer activity. While describing arachidin-1, this reference does not describe treatment or prevention of rotavirus infections.

Sobolev, V. S. et al. (2011) Biological Activity of Peanut (Arachis hypogaea) Phytoalexins and Selected Natural and Synthetic Stilbenoids, J. Agric. Food Chem. 59(5), 1673-1682 [3]. This reference describes the production of stilbenes compounds by a peanut plant as induced as a response to a microbial pathogen. The reference investigates peanut phytoalexins, together with some related natural and synthetic stilbenoids (a total of 24 compounds) in a panel of bioassays to determine their anti-inflammatory, cytotoxic, and antioxidant activities in mammalian cells. This included both arachidin-1 and arachidin-3. The highest but moderate cytotoxicity was exhibited in all cell lines by arachidin-1. While describing arachidin-1, this reference does not describe treatment or prevention of rotavirus infections.

### 2. DETAILED DESCRIPTION

Rotavirus (RV) infections are the most common cause of severe viral diarrhea in infants and young children worldwide. RV vaccines are available, but are cost prohibitive for many and only reduce severe viral symptoms. Vaccinated infants continue to shed infectious virus particles and contribute to RV spread. Recent studies have voiced concerns about the efficacy of the current RV vaccines against new emerging RV strains in developing countries. Continuing surveillance to identify new RV strains, assessing vaccine efficacy, and developing new therapeutic agents to treat RV disease need to be pursued and provided at an affordable cost. The purpose of the study contained within was to determine the efficacy of stilbenoid compounds, which have antioxidant and anti-inflammatory properties to inhibit RV replication and/or pathology. Peanut *(A. hypogaea)* hairy root cultures were induced to produce stilbenoids, which were purified by high performance counter current chromatography (HPCCC) and analyzed by HPLC. Different concentrations of the stilbenoids and RV were added to HT29.f8 cells, and incubated for 12 and 24 hours post-infection. The cells and supernatants were collected and viral titers were calculated and compared to determine the effects of the stilbenoid treatment. Two of the four stilbenoids tested, *trans*-arachidin-1 and *trans*-arachidin-3, showed a significant decrease in RV titers at 24 hours post-infection. Western blot analyses performed on the infected cell lysates complemented the titers and indicated a significant decrease in viral replication. These data show stilbenoids may be effective as a RV theurapeutic.

In one embodiment, the current invention contemplates a prenylated stilbenoid for use in a method comprising a host (human and animal)-oriented antiviral treatment that can affect a wide-range of rotavirus (RV) strains including, but not limited to, human rotavirus Wa, simian rotavirus SA114F, porcine rotavirus OSU, bovine rotavirus 2292B and equine rotativirus H2, and restores the hosts' ability to reduce the disease burden of rotavirus infections. There are many other rotavirus strains but these represent some well genome-sequenced and referenced strains. The present invention also encompasses rotaviruses formed from newly reassorted viruses that include parts of their genome from animal as well as human rotaviruses

In particular, prenylated stilbenoids (i.e., for example, arachidin-1 and arachidin-3) may be combined with naturally occurring antioxidants and/or anti-inflammatory compounds. It is suggested that prenylated stilbenoids have improved bioavailability because the prenyl group can block access to the glucuronidation sites in the stilbenoid structure. In addition, prenylated stilbenoids, but not non-prenylated stilbenoids, can modulate cannabinoid receptors which could alter cellular metabolism in a way that is unfavorable for viral replication. Purified prenylated stilbenoids were tested in antiviral assays. These experiments showed significant differences of the production of infectious virus particles and Non-Structural Protein 4 (NSP4) at 24 hours post infection and 20 µM concentrations of two of the four tested stilbenoids, arachidin-1 and arachidin-3.

### INTRODUCTION:

The mechanism(s) of RV-induced diarrhea is multifactorial, and include both secretory and maladsorbtive diarrhea phases. Despite much effort, a complete understanding of RV pathophysiology is not known [4]. Most vaccine strategies to deter RV-induced diarrhea are designed to arm the immune system using either attenuated live RV or RV proteins [5]. There are two licensed rotavirus (RV) vaccines in the United States, RotaTeq®, produced by Merck and Rotarix® produced by GlaxoSmithKline. Both are effective in preventing severe diarrhea in vaccinated children [6, 7]. The vaccines are designed to protect against common RV strains and therefore are dependent on the genetic stability of the virus. Reassortment events of the RV segmented genome are common and lead to new virulent RV strains that may not be averted by the current vaccines. New reassorted RV strains frequently are discovered worldwide [8]. Likewise, the zoonotic nature of RV infections supports the argument to continue to survey for emerging RV strains arising from interspecies transmission with potential of vaccine failures [9]. Furthermore, high costs, limited availability, and poor logistics for the distribution of the vaccines are challenging problems for the developing world [6]. Consequently, the development of cost effective, easily distributed, novel, and host-oriented antiviral paradigms are needed that affect a wide-range of RV strains and reduce the disease burden of RV infections. One major embodiment of the current invention takes advantage of the antioxidant and anti-inflammatory properties of a natural product to treat RV infections meets the principles of a novel therapeutic strategy.

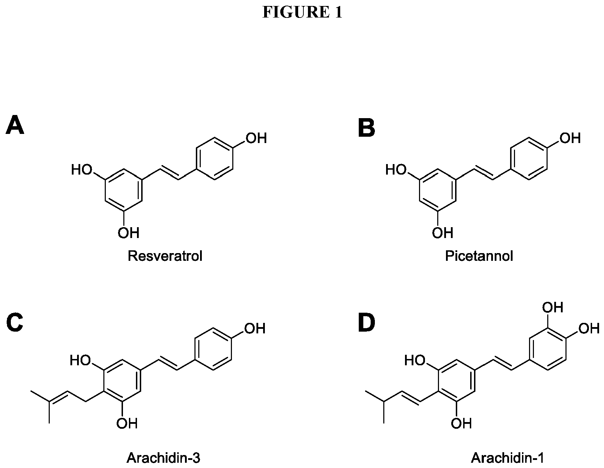

Stilbenoids are phenolic compounds derived from the phenylpropanoid/acetate pathway. Among these compounds, resveratrol and its natural analogs exhibit antioxidant, anti-inflammatory and anti-cancer properties that benefit human health [3]. *Trans*-resveratrol (t-Res) has been an extensively studied stillbenoid and demonstrates strong antioxidant and chemo-preventive properties [3]. Stilbenoids are produced by a group of plants which includes, but is not limited to, grapes, peanuts and some berries [3, 10, 11]. *Trans*-Piceatannol (t-PA) is a hydroxylated analog of resveratrol found in grapes and in minor quantities in peanuts. *Trans*-Arachidin-1 (t-A1) is a prenylated (3-methyl-1-butenyl) analog of piceatannol, whereas *trans*-arachidin-3 (t-A3) is a prenylated (3-methyl-1-butenyl) analog of resveratrol (Figure 1A-D). Both t-A1 and t-A3 are produced in peanuts upon fungal challenge. These stilbenoids can be extracted from some plants, but are not suitable for many applications in the food/pharmaceutical sectors due to the overall low concentration of stilbenoids in the plant extracts. To deliver a highly defined and stilbenoid-enriched product, hairy root cultures of peanut (*A. hypogaea*) have been established in a bioproduction system that produces increased levels of stilbenoids, including t-A1 and t-A3, upon treatment with elicitors, [1]. t-PA and t-Res are commercially available, but t-A1 and t-A3 are still in an experimental stage resulting in an opportunity to explore new antiviral biological activity.

The therapeutic potential of four exemplary stilbenoids, t-Res, t-PA, t-A1 and t-A3 (Figure 1A-D), to inhibit RV infections in culture using a cloned human intestinal cell line, HT29.f8 was assessed [12]. Not limiting the current invention to any specific mechanism, the hypothesis for this invention is that stilbenoids will modulate the viral load of RV generated during an infection. Two sets of experiments were performed in which different concentrations of the stilbenoids and two time points post infection were evaluated. To determine the effect of the stilbenoids on the amount of virus produced during an infection, viral titers were determined using the supernatants for each of the different treatments (10 µM and 20 µM stilbenoids) collected at 12 and 24 hours post infection (hpi). The viral titers produced in cells treated with the stilbenoids were compared to the virus titers generated from RV infections alone, and reported as fold changes in the production of infectious virus particles. Western blot analyses using the corresponding cells demonstrated the presence of a nonstructural RV protein (NSP4 nonstructural protein 4) to verify infection.

### RESULTS:

### Bioproduction of stilbenoids in hairy root cultures of peanut

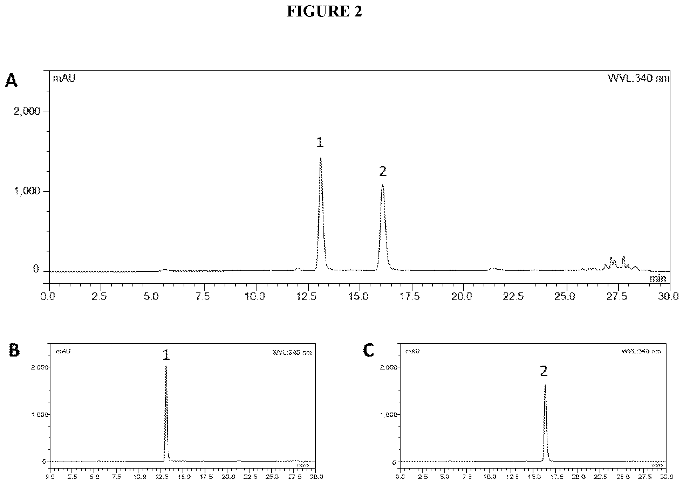

To produce the stilbenoids t-A1 and t-A3, previously established hairy root line 3 from peanut cv. Hull were used. These hairy roots are capable of synthesizing and secreting t-Res, t-A1 and t-A3 into the culture medium upon treatment with the elicitor sodium acetate [1]. Depending on the period of elicitor treatment, the levels and types of stilbenoids found in the medium can be modified [1]. To study the effect of other elicitors on production of t-A1 and t-A3 different elicitors were tested, including, but not limited to, methyl-β-cyclodextrin (CD). In preliminary experiments, different doses of CD were added to the hairy root cultures for different periods between 0 and 96 h (data not shown) and a 72 h treatment of 9 g/L CD was selected based on the highest levels of t-A1. As shown in Figure 2A-C, t-A1 and t-A3 were the major stilbenoids present in the culture medium. T-Res was present in very small amounts in these extracts. To purify t-A1 and t-A3, ethyl acetate extracts were made from the culture medium and subjected to HPCCC (high performance counter current chromatography). The solvent system was adapted from a previously used CPC (centrifugal partition chromatography) system which was effective in purifying t-A1 and t-A3 from hairy root culture medium extracts [10]. The only modifications were the replacement of heptane for hexane and ethanol for methanol. The separation was effective and comparable to the one achieved before [10]. Thus, high yields of highly purified fractions of t-A1 and t-A3 were achieved and were used in the antiviral assays.

### Viability of HT29.F8 cells in the presence of 0.02% DMSO

The percentage of live/dead cells was calculated using the trypan blue exclusion dye assay (Table 1). At 12 hours post-inection (hpi), uninfected HT29.f8 cells with and without 0.02% DMSO showed a cell viability of 93% and 94%, respectively (Table 1). RV alone showed a viability of 80% demonstrating a viral effect on the cells, but RV with 0.02% DMSO showed 85% cell viability. At 24 hours post-infection, uninfected HT29.f8 cells containing 0.02% DMSO showed a viability of 93%. Both RV alone and RV with 0.02% DMSO showed a viability of 85% demonstrating a viral effect on the cells (Table 1). These data revealed that the addition of RV does increase cell death, but the addition of DMSO to the culture system, did not adversely affect the viability of the HT29.f8 cells in culture or diminish viral replication.

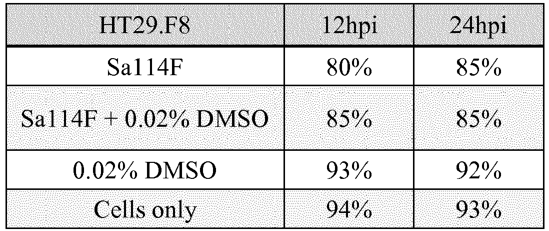

Table 1 shows the Viability of HT29.f8 cells with/without RV and with/without DMSO at 12 and 24 hours post RV infection.

### The effects of stilbenoids on the production of infectious rotavirus particles

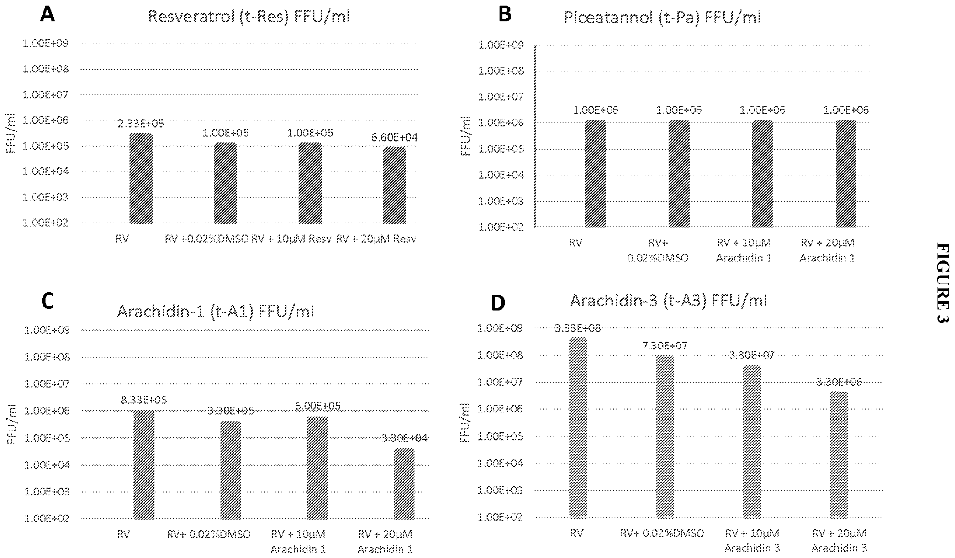

Viral titers were determined using FFU assays from the supernatants of RV-infected HT29.f8 cells treated with exemplary stilbenoids (10 µM and 20 µM; t-Res, t-PA, t-A1, or-A3). Supernatants collected at 12 hours post-infection were equivalent to the RV-infected control cells (data not shown). Likewise, viral titers at 24 hours post-infection with 10 µM concentrations of all the stilbenoid treated samples showed no difference from the RV-infected cells (data not shown). Similarly, at 24 hours post-infection, the 20 µM concentrations of the non-prenylated stilbenoids, t-Res and t-PA, both demonstrated no change in the virus titer when compared to the RV-infected control (Figure 3A&B). However at 24 hours post-infection, the 20 µM concentrations of the two prenylated stilbenoids, t-A1 and t-A3, each generated a one hundred fold decrease in virus titer when compared to the RV-infected control supernatants (Figure 3C&D).

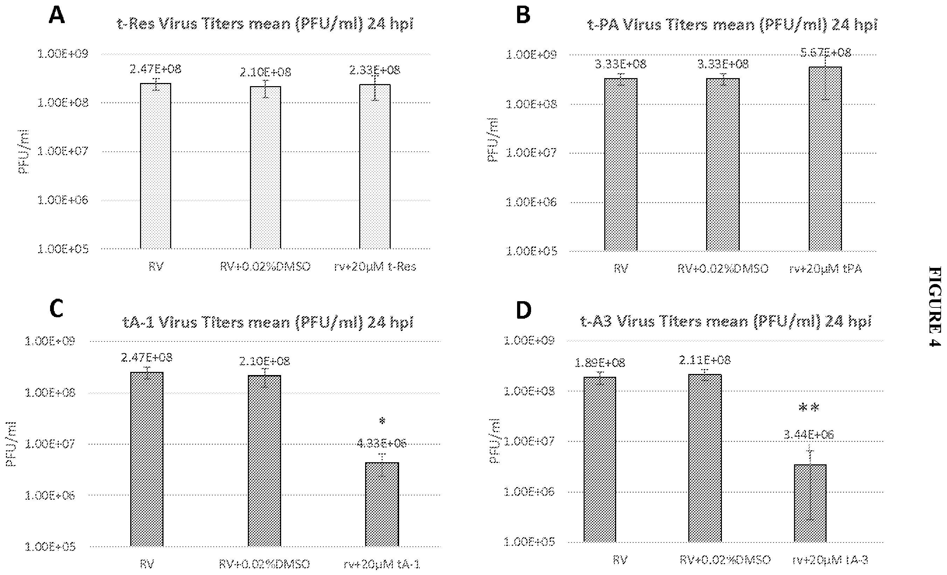

The same supernatants that were used for the FFU assays were utilized for the plaque assays. Using the same 20 µM t-Res, t-PA, t-A1 and t-A-3, plaques were counted and the average of three experiments was calculated and graphed as PFU/ml (Figure 4A-D).

The data produced using the PFU assay showed the same fold differences as shown with the FFU assays (Figure 4A-D). Using the student-T test, the average and standard deviations were calculated and graphed (Figure 4A-D). The experiments using t-PA and t-Res showed no statistical differences between RV only and RV with DMSO. However, the experimental data from t-A1 and t-A3 PFU assays demonstrated a one hundred fold difference that was statistically different from RV only and RV with DMSO for t-A1 or t-A3 (p≤ 0.04, and 0.02, respectively).

### Western blot (WB) analyses implies differences in RV replication

To complement and visualize the differences demonstrated in the viral titers between RV alone, RV with DMSO, and 20 µM t-A1, western blot assays were performed as previously described [13-15]. Using equal amounts of protein of the corresponding cell lysates, the nonstructural viral protein 4, NSP4 was detected in all RV-infected cell lysates. The western blot data of the RV and RV with DMSO both demonstrated relatively equal amounts of multimeric forms, di-glycosylated (fully glycosylated), mono-glycosylated, and cleavage fragments of NSP4 (Figure 5, lanes 1 and 2.). The results for RV with t-A1 displayed only the fully glycosylated form of NSP4 (Figure 5). This is commonly observed when the concentration of NSP4 is small (data not shown). This indicates viral replication is negatively affected by 20 µM t-A1. Lane 4, (Figure 5) reveals no bands and shows the specificity of the anti-NSP4 antibodies.

### DISCUSSION

The data presented herein shows a dose- and time-dependent decrease in viral progeny (one hundred fold) when RV and prenylated stilbenoids (t-A1 or t-A3) were incubated with the human intestinal cell line HT29.F8. The presence of the nonstructural viral protein NSP4 in the western blot assays confirms the RV infection and indicates the virus was replicating in the HT29.f8 cells. This is the first study performed with a RV-infected human intestinal cell line testing the effects of stilbenoids, t-Res, t-PA, t-A1 and t-A3 on virus production.

The prenylated stilbenoids, t-A1 and t-A3, are significantly more lipophilic than either of the non-prenylated t-Res or t-PA molecules. It is believed that the prenylated side chain increases the lipophilicity of the molecules to which it is attached. Consequently, prenylation is thought to promote association with and penetration through cell membranes. An increase in lipophilicity often correlates positively with increased biological activity within different groups of compounds of similar structure [16, 17]. Several delivery systems including emulsions and nanoparticles have been tested for the delivery of lipophilic, bioactive natural products [18]. Depending on the application, these delivery systems may be applicable to t-A1 and t-A3 as potential therapeutic agents.

Although the molecular mechanism(s) for the protective effect of t-A1 and t-A3 are not known, the inhibition of viral replication may be attributed to the anti-oxidative and anti-inflammatory properties of the constituent stilbenoids. Recently t-A1 and t-A3 have been shown to modulate the cannabinoid receptors at micromolar levels [18, 19]. The experimental data disclosed herein indicates that t-A3 may act as a competitive cannabinoid receptor 1 (CB1R) antagonist, whereas t-A1 may antagonize CB1R agonists by both competitive and non-competitive mechanisms [19]. The HT29 cell line, the parent cell line of HT29.f8, expresses cannabinoid receptors [20]. These receptors are part of the endocannabinoid signaling system, and it is well known that the endogenous cannabinoid system and cannabinoid receptors regulate gastrointestinal functions, such as gastric emptying, secretion, and intestinal motility [21, 22]. Additionally, many studies demonstrate the importance of the cannabinoid receptors in the regulation of different types of cancers through diverse cellular pathways [23]. In a study on colorectal cancer, cannabinoid receptor (CB1 and CB2) agonists were shown to have an effect on apoptosis through TNFα-mediated increase in ceramide production [24]. Another study on breast cancer shows the receptor agonists inhibit adenylyl cyclase activity, cAMP, and PKA activity resulting in the down regulation of gene transcription [25, 26]. In another study, a cAMP-dependent PKA mechanism may be important in RV pathogenesis in a human intestinal cell line, Caco2. [27].

Recently, cannabinoid receptors were proposed as potential therapeutic agents against hepatitis C virus by modulating lipid homeostasis [28]. A study by Gaunt *et al.* (2013) using RV-infected MA104 cells demonstrate a dose-dependent reduction in virus infectivity and viral RNA production with the addition of TOFA [5-(tetradecyloxy)-2-furoic acid], an inhibitor of the fatty acid synthase enzyme complex [29]. Further, the infectivity of RV in ACC1 knockdown cells was reduced by 8.5-fold (significant, p=0.01) with siRNA directed against ACC1, the gene that encodes the enzyme catalyzing the rate-limiting step of the palmitoyl-CoA synthetic pathway. This strongly suggests that RV infectivity may be mediated through fatty acid metabolism [29] or selected fatty acids.

Altogether, these data imply a possible antiviral mechanism for t-A1 and t-A3 through modulation of the cannabinoid receptors and subsequent alteration of fatty acid metabolism in the host cell. Thus, in one embodiment, t-A1 and/or t-A3 are used to design and develop more efficacious RV therapeutic agents.

### EXPERIMENTAL

### Example I

### Cells, virus, and reagents

The objective of the study was to test the effect(s) of four exemplary stilbenoids on RV replication in HT29.F8 cells with variable concentrations of the stilbenoids and different collection times. Based on previous studies that assessed the effect of different amounts of stilbenoids and DMSO on Influenza A and polyomaviruses, respectively [30, 31], 10 µM and 20 µM concentrations of each stilbenoid were tested at 12 and 24 hours post-infection. A total of five experimental sets were performed per stilbenoid. In the first experimental set, cells were infected with SA114F RV at a multiplicity of infection (MOI) of 2 as previously reported [14]. In the second experimental set, 0.02% DMSO was added to the RV infection to prove that 0.02% DMSO used to solubilize the stilbenoids had no effect on cell viability or production of RV. In the third and fourth experimental sets, 10 µM or 20 µM concentrations of the stilbenoids, respectively, were solublized in 0.02% DMSO in DMEM, added to the RV inoculum, and used to infect the cells. The fifth set was uninfected HT29.f8 cells. Each experimental set was tested in four wells of a 24 well tissue culture (TC) plate. The media from the four wells were pooled, centrifuged and the supernatants were stored at -80 °C and used to determine viral titers. The cells were collected in PBS, frozen and thawed 3 times, and centrifuged. The supernatants were collected as the cell lysates and stored at -80 °C until used in western blot assays. Viral titers were performed in triplicate by both focus forming units (FFU) and plaque forming units (PFU). Equal amounts of the cell lysates were used in western blot assays to resolve and probe for RV NSP4.

### Example II

### Bioproduction and purification of the stillbenoids

Hairy roots of peanut cv. Hull (line 3) were cultured in 250 ml flasks containing 50 ml of MSV medium as previously described [1, 32]. At day nine of culture, the spent medium was removed and replaced with fresh MSV medium containing 9 g/L methyl-β-cyclodextrin (Cavasol® W7 M) and incubated in the dark at 28°C for an additional 72 h to induce synthesis and secretion of stilbenoids into the culture medium. The medium from each flask was pooled and partitioned with ethyl acetate to extract the stilbenoids. The ethyl acetate extract was dried in a rotavapor (Buchi) and t-A1 and t-A3 were purified from the extract by HPCCC as follows. The dried ethyl acetate extract was resuspended in HPCCC solvent system (hexane:ethyl acetate:methanol:water [4:5:3:3]) and injected into a Spectrum™ (Dynamic Extractions) HPCCC system. The upper phase of the solvent system was used as stationary phase and the chromatography was monitored at UV 340 nm. Fractions were collected every 30 s, dried in a speed-vac and analyzed by HPLC.

HPLC analyses were performed in a Dionex Summit system, equipped with a photodiode array (PDA) detector. The separation was performed on a SunFire™ C₁₈, 5 µm, 4.6 x 250 mm column (Waters) at 40 °C with a flow rate of 1.0 ml/min. The mobile phase consisted of 2% formic acid in water (A) and methanol (B). The method started with 100% A for 1 min. Then a linear gradient was performed from 60% A and 40% B to 65% A and 35% B (1 to 20 min), followed by a linear gradient from 65% A and 35% B to 100% B. Then the column was washed with 100% B for 5 min (20 to 25 min). Elicited peanut seed-derived t-A1 and t-A3 were used as reference standards [32].

Purity of the fractions was monitored by HPLC using UV absorbance at 280, 320, and 340 nm. Selected fractions were also checked for purity by mass spectrometry using an UltiMate 3000 ultrahigh performance liquid chromatography (UHPLC) system (Dionex, Thermo Scientific) coupled with a LTQ XL linear ion trap mass spectrometer (Thermo Scientific) as described in Marsh *et al.* (2014). HPCCC fractions containing t-A1 and t-A3 with over 95% purity based on HPLC analysis (UV 340 nm) were combined, dried under a nitrogen stream and used for viral assays. The dry mass of the purified stilbenoids were reconstituted in 0.02% dimethyl sulfoxide (DMSO) in DMEM medium with 1 µg/m trypsin. To compare the results between non-prenylated stilbenoids (t-Res and t-PA) and their prenylated analogs (t-A3 and t-A1, respectively) the synthetic/commercially available t-Res (Sigma-Aldrich) and t-PA (Alexis) were used in this study.

### Example III

### Cell lines and virus

MA104 cells were obtained from ATCC (Rockville, MD) and the HT29.F8 cells, a spontaneously polarizing cell line, were derived from the parent human adenocarcinoma (HT29) intestinal line [12]. The cell lines were confirmed to be free of mycoplasma contamination using the MycoFind mycoplasma PCR kit version 2.0 (Clongen Laboratories, LLC)

RV SA11 clone 4F [33] was grown and titered in MA104 cells and stored at -80 °C.

### Example IV

### Viability Assay

The percent live/dead cells was calculated using the trypan blue dye exclusion assay as previously outlined [34]. Briefly, a cell suspension of ∼ 10⁶ cells/ml was diluted 1:1 with a 0.4% trypan blue solution, and loaded onto a hemocytometer. The number of stained cells and total number of cells were counted, and the calculated percentage of unstained cells was reported as the percentage of viable cells. To determine if the 0.02% DMSO that was used to solubilize the hydrophobic stilbenoids adversely effected the life span of HT29.F8 cells, viability assays were performed with RV alone, RV with 0.02% DMSO, cells with 0.02% DMSO and cells alone using the trypan blue cell exclusion assay as described [34].

### Example V

### Virus quantification

To test the biological activity of the stilbenoids on RV infections, both FFU and PFU assays were performed as previously described [35]. MA104 cells were grown to 80% confluence in 24 well tissue culture plates (Corning Life Sciences); starved for fetal bovine sera 12 h prior to infection; and then infected with RV SA114F. Briefly, the SA114F RV stock was sonicated (5 min using a cuphorn attachment and ice bath in a Misonix Sonicator 3000; Misonix, Inc, Farmingdale, NY) and incubated in serum-free DMEM with 1 µg/ml trypsin (Worthington Biochemical, Lakewood, NJ) for 30 min at 37°C. The activated viral inoculum was added to the cells for 1 h at 37°C in 5% CO₂ at an MOI of 2. The inoculum was replaced with serum-free DMEM supplemented with 1 µg/ml trypsin and incubated for 12 and 24 hours post-infection. The cell lysates were collected, subjected to repeated freeze-thaws, clarified at 850 x g for 5 min. Media was collected, clarified at 850 x g for 5 min, and stored at -80°C. Both the cell lysates and supernatants were stored at -80°C. Viral titers were done in triplicate by indirect immunofluorescent staining of MA104 monolayers infected with serial dilutions of the supernatants. The average number of fluorescent foci was calculated for three wells and used to determine the number of focus forming units/ml (FFU/ml) [36]. To complement the FFU assays, plaque forming assays were performed in triplicate as outlined above for the FFU assays, except after the 1 hour infection, the virus inoculum was replaced with 3mls of a medium overlay (1:1 mixture of 1.2% agarose and complete 2 × MEM containing 0.5µg/ml trypsin) and incubated at 37°C in 5% CO₂ for 3 to 4 days or until plaques are visible. A neutral red overlay (1:1 mixture of 1.2% agarose with an equal volume of serum-free 2× MEM containing 50 µg/ml neutral red) was prepared and 2 ml per well of stain overlay was added on top of the first agarose/medium overlay. The six well plates were incubated at 37°C until plaques were visible (approximately 4 to 24 h). The individual plaques were counted and the titer was calculated as follows: Number of plaques x 1/dilution factor x 1/(ml of inoculum) = PFU/ml.

### Example VI

### Protein quantification and Western Blot assays

The micro bicinchoninic acid (BCA) protein assay was employed to quantify protein concentrations using bovine serum albumin as the standard per manufacturer's protocol (Thermo Scientific Pierce). One microgram of total protein from each sample was separated by 12.5% SDS-PAGE, electroblotted onto nitrocellulose membranes, probed with NSP4 peptide-specific antibodies [37, 38] and reactive bands visualized by the addition of HRP-conjugated IgG and Super Signal® West Pico chemiluminescent substrate (Pierce) followed by exposure to Kodak X-OMAT film [13-15].

### Example VII

### Observations of cell ultrastructure with TEM of

### rotavirus (RV) infected HT29.f8 cells w/without t-A3

### RV(Wa) only

1. Autophagic vesicles (AV) that are present when the autophagic pathway to cell death is in progress
2. Very few RV particles present in the cells at 18hpi
3. Few mitochondria present that appeared to be swollen and much larger than normal
4. Many vesicles present in the cytoplasm
5. Enlarged nucleus with no nucleoli present

### RV(Wa) with 20 µM arachidin-3

1. Autophagic vesicles (AV) absent
2. Many RV particles present in cells at 18hpi, about 50% observed were noninfectious, immature enveloped particles
3. Many mitochondria present with normal ultrastructure
4. Vesicles present in the cytoplasm but not as many as in the RV(Wa) only
5. Nucleoli present

Autophagic vesicles are present in rotavirus infected cells. However, autophagic vesicles are absent in rotavirus infected cells treated with arachidin-3.

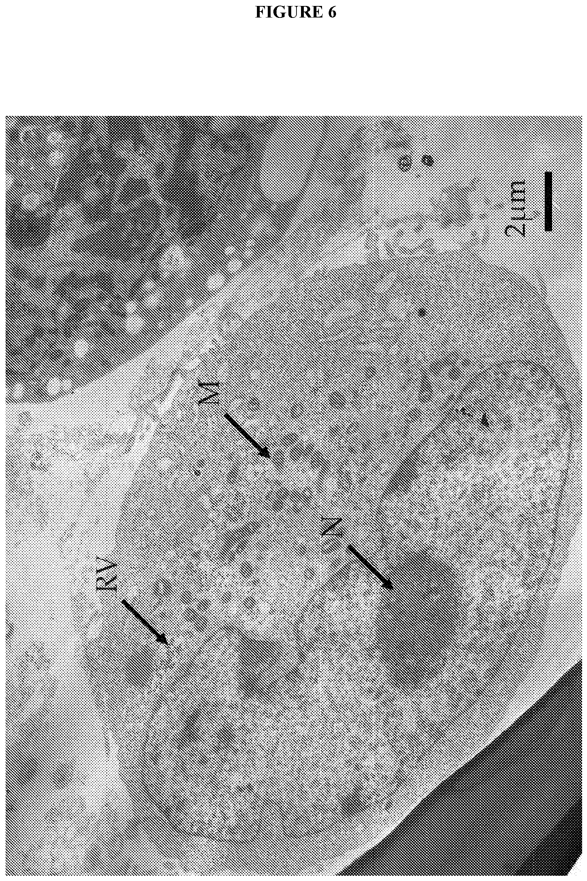

Figure 6 shows a transmission electron micrograph shows rotavirus (Wa) infected HT29.f8 with 20 mM arachidin-3 at 18 hours post infection. Mitochondria (M), rotavirus particles (RV), and a nucleolus (N) can be seen. Final Magnification = 8,300X.

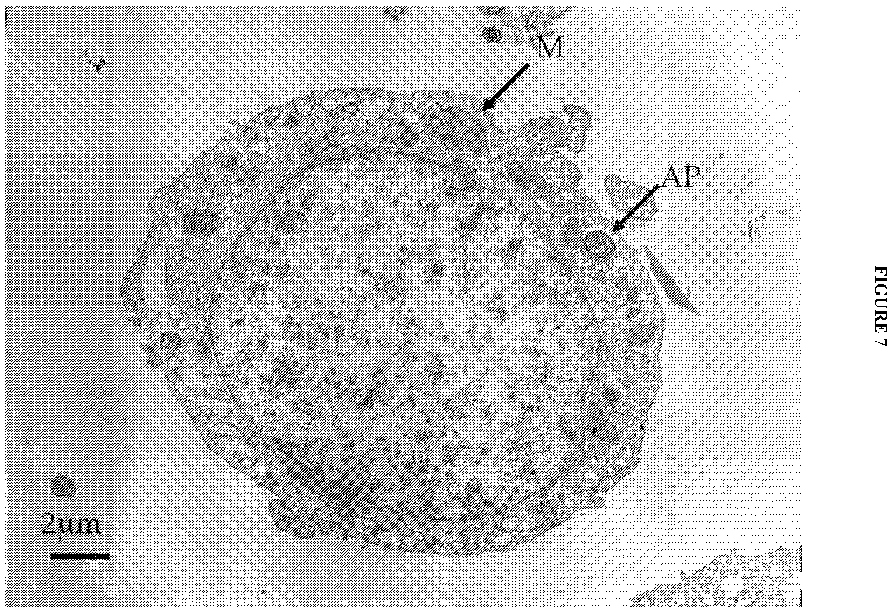

Figure 7 shows a transmission electron micrograph shows rotavirus (Wa) infected HT29.f8 cells 18 hours post infection. Mitochondria (M) and autophagosomes (AP) can be seen. Final Magnification = 7,800X

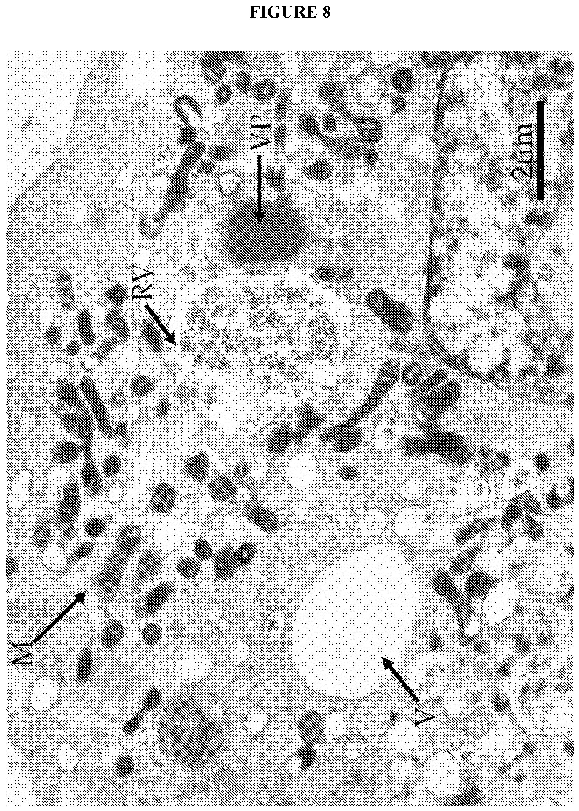

Figure 8 shows a transmission electron micrograph shows rotavirus (Wa) infected HT29.f8 cells with 20 mM arachidin-3 at 18 hours post infection. Mitochondria (M) and vacuoles (V) can be seen. A Viroplasm (VP) can be seen where rotavirus (RV) particles are produced. Final Magnification = 10,000X

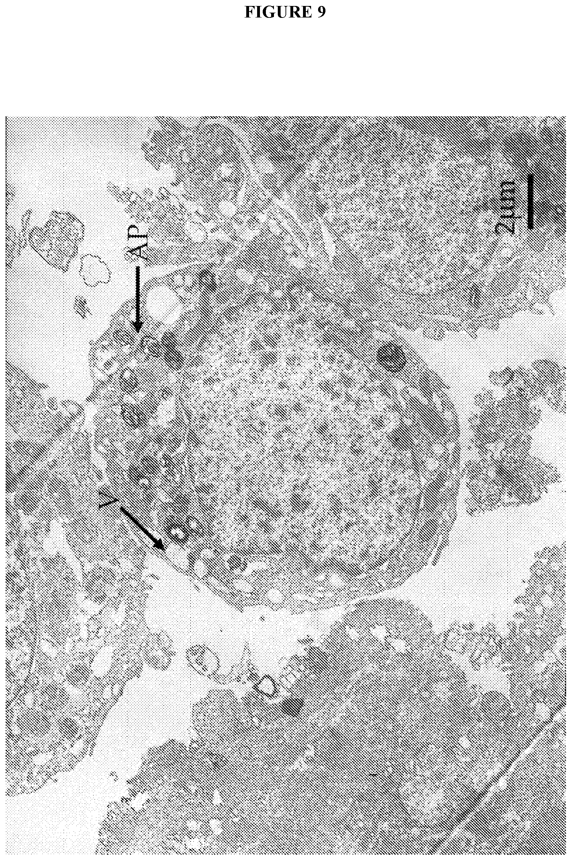

Figure 9 shows a transmission electron micrograph shows rotavirus (Wa) infected HT29.f8 cells 18 hours post infection. Autophagosomes (AP) and vacuoles (V) can be seen. Final Magnification = 7,600X

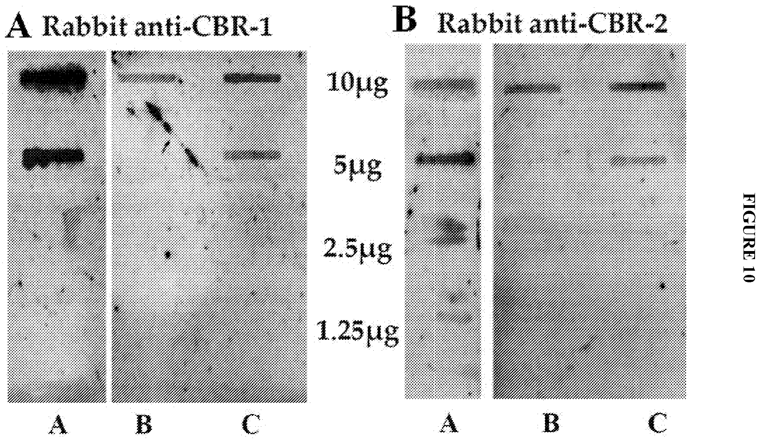

Figure 10A&B shows cell lysates (Figure 10A -uninfected HT29.f8; Figure 10B-uninfected MA104); C-RV-infected (Wa) MA104/HT29.f8 cell lysates were added to nitrocellulose membranes, probed with a 1:1000 dilution of rabbit anti-CNR1/2 antibodies from Antibodies Online (Atlanta, GA) and reactive bands were visualized by the addition and excitation of goat anti-rabbit antibodies Alexa Fluor®546 (Life Technologies) using the Typhoon 8600 laser scanner.

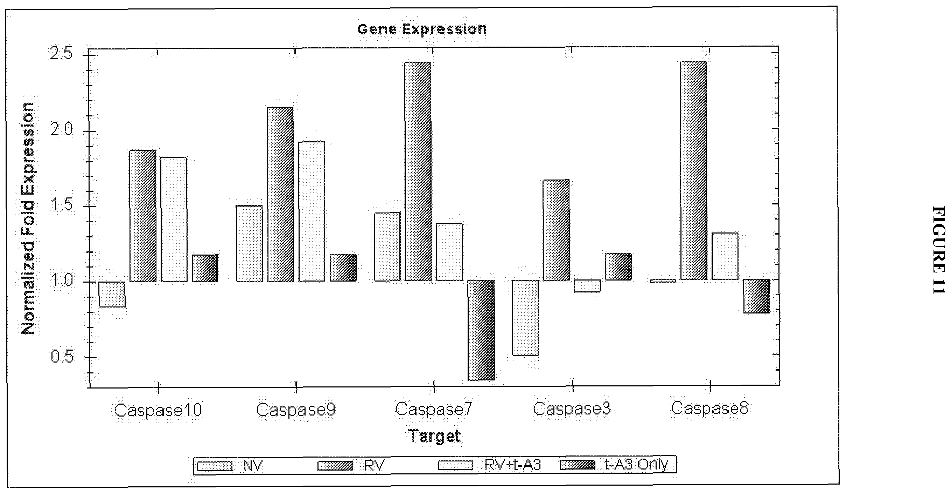

Figure 11 shows the fold change in caspase genes expression. Total RNA was extracted at 8 hours post RV infection of HT29.f8 cells with/without arachidin-3 and controls of no virus and arachidin-3 only. Using qRT-PCR, the cDNA was amplified using gene-specific primers to detect caspases 3, 7, 8, 9, & 10 transcripts and normalized with human GAPDH and b-actin. The experiment were performed in duplicate. The green boxes show the results of the caspases that are effected by arachidin-3. The ΔCt values obtained from the qRT-PCR were used to determine the relative fold changes in gene expression.
NV- no virus + 0.0002% DMSO
RV- human rotavirus (Wa strain) only + 0.0002% DMSO
RV+arachidin-3- human rotavirus (Wa strain) with 20 mM arachidin-3
t-arachidin-3- 20 mM arachidin-3 only

These studies have shown that the antiviral (rotavirus) mechanism is by inhibition of autophagy. As mentioned below, many viruses exploit autophagy to promote viral replication. This includes important RNA viruses such as picornaviruses (poliovirus, rhinovirus, hepatitis A), coronaviruses, flaviviruses (hepatitis C, yellow fever, dengue fever, dengue virus and west nile virus). There is also information about zika virus and autophagy. It is believed that the prenylated stilbenods may have a similar effect because these other viruses use a similar mechanism to promote their replication.

The present invention, therapeutic applications of prenylated stilbenoids against rotavirus infections, is well adapted to attain the ends and advantages mentioned as well as those that are inherent therein. The particular embodiments disclosed above are illustrative only, as the present invention may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered or modified and all such variations are considered within the scope and spirit of the present invention. Also, the terms in the claims have their plain, ordinary meaning unless otherwise explicitly and clearly defined by the patentee. Although the invention has been described with reference to these preferred embodiments, other embodiments can achieve the same results. Variations and modifications of the present invention will be obvious to those skilled in the art and it is intended to cover in the appended claims all such modifications and equivalents. The entire disclosures of all applications, patents, and publications cited above, and of the corresponding application are hereby incorporated by reference.

### REFERENCES:

1. Condori, J. et al. (2010) "Induced Biosynthesis of Resveratrol and the Prenylated Stilbenoids Arachidin-1 and Arachidin-3 in Hairy Root Cultures of Peanut: Effects of Culture Medium and Growth Stage," Plant Physiol. Biochem. 48(5), 310-318.
2. Huang, C.-P. et al. (2010) "Arachidin-1, a Peanut Stilbenoid, Induces Programmed Cell Death in Human Leukemia Hl-60 Cells," J. Agric. Food Chem. 58(23), 12123-12129.
3. Sobolev, V. S. et al. (2011) "Biological Activity of Peanut (Arachis Hypogaea) Phytoalexins and Selected Natural and Synthetic Stilbenoids," J. Agric. Food Chem. 59(5), 1673-1682.
4. Desselberger, U. (2014) "Rotaviruses," Virus Res. 190(0), 75-96.
5. Tate, J. E. and Parashar, U. D. (2014) "Rotavirus Vaccines in Routine Use," Clin. Infect. Dis. 59(9), 1291-1301.
6. Leshem, E. et al. (2014) "Distribution of Rotavirus Strains and Strain-Specific Effectiveness of the Rotavirus Vaccine after Its Introduction: A Systematic Review and Meta-Analysis," Lancet Infect. Dis. 14(9), 847-856.
7. Yen, C. et al. (2014) "Rotavirus Vaccines," Hum. Vaccin. Immunother. 10(6), 1436-1448.
8. Weinberg, G. A. et al. (2013) "Detection of Novel Rotavirus Strain by Vaccine Postlicensure Surveillance," Emerg. Infect. Dis. 19(8), 1321.
9. Martinez, M. et al. (2014) "Whole-Genome Analyses Reveals the Animal Origin of a Rotavirus G4p[6] Detected in a Child with Severe Diarrhea," Infect. Genet. Evol. 27(0), 156-162.
10. Abbott, J. A. et al. (2010) "Purification of Resveratrol, Arachidin-1, and Arachidin-3 from Hairy Root Cultures of Peanut (Arachis Hypogaea) and Determination of Their Antioxidant Activity and Cytotoxicity," Biotechnol. Prog. 26(5), 1344-1351.
11. Michel, T. et al. (2013) "New Concepts, Experimental Approaches, and Dereplication Strategies for the Discovery of Novel Phytoestrogens from Natural Sources," Planta Med. 79(07), 514-532.
12. Mitchell, D. M. and Ball, J. M. (2004) "Characterization of a Spontaneously Polarizing Ht-29 Cell Line, Ht-29/Cl.F8," In Vitro Cell. Dev. Biol. Anim. 40(10), 297-302.
13. Gibbons, T. F. et al. (2011) "Rotavirus Nsp4: Cell Type-Dependent Transport Kinetics to the Exofacial Plasma Membrane and Release from Intact Infected Cells," Virol. J. 8, 278.
14. Parr, R. D. et al. (2006) "The Rotavirus Enterotoxin Nsp4 Directly Interacts with the Caveolar Structural Protein Caveolin-1," J. Virol. 80(6), 2842-2854.
15. Storey, S. M. et al. (2007) "Full-Length, Glycosylated Nsp4 Is Localized to Plasma Membrane Caveolae by a Novel Raft Isolation Technique," J. Virol. 81(11), 5472-5483.
16. Schultz, T. et al. (1997) "Quantitative Structure-Activity Relationships of Stilbenes and Related Derivatives against Wood-Destroying Fungi," Recent Res. Devel. Agric. Food Chem. 1, 289-299.
17. Yazaki, K. et al. (2009) "Prenylation of Aromatic Compounds, a Key Diversification of Plant Secondary Metabolites," Phytochemistry 70(15-16), 1739-1745.
18. Silva, F. et al. (2014) "Strategies to Improve the Solubility and Stability of Stilbene Antioxidants: A Comparative Study between Cyclodextrins and Bile Acids," Food Chem. 145(0), 115-125.
19. Brents, L. K. et al. (2012) "Natural Prenylated Resveratrol Analogs Arachidin-1 and -3 Demonstrate Improved Glucuronidation Profiles and Have Affinity for Cannabinoid Receptors," Xenobiotica 42(2), 139-156.
20. Ihenetu, K. et al. (2003) "Inhibition of Interleukin-8 Release in the Human Colonic Epithelial Cell Line Ht-29 by Cannabinoids," Eur. J. Pharmacol. 458(1-2), 207-215.
21. Coutts, A. A. and Izzo, A. A. (2004) "The Gastrointestinal Pharmacology of Cannabinoids: An Update," Curr. Opin. Pharmacol. 4(6), 572-579.
22. Massa, F. and Monory, K. (2006) "Endocannabinoids and the Gastrointestinal Tract," J. Endocrinol. Invest. 29(3 Suppl), 47-57.
23. Pisanti, S. et al. (2013) "The Endocannabinoid Signaling System in Cancer," Trends Pharmacol. Sci. 34(5), 273-282.
24. Cianchi, F. et al. (2008) "Cannabinoid Receptor Activation Induces Apoptosis through Tumor Necrosis Factor α-Mediated Ceramide De Novo Synthesis in Colon Cancer Cells," Clin. Cancer Res. 14(23), 7691-7700.
25. Bifulco, M. et al. (2008) "Endocannabinoids in Endocrine and Related Tumours," Endocr. Relat. Cancer 15(2), 391-408.
26. Laezza, C. et al. (2010) "Inhibition of 3-Hydroxy-3-Methylglutaryl-Coenzyme a Reductase Activity and of Ras Farnesylation Mediate Antitumor Effects of Anandamide in Human Breast Cancer Cells," Endocr. Relat. Cancer 17(2), 495-503.
27. Martin-Latil, S. et al. (2004) "A Cyclic AMP Protein Kinase a-Dependent Mechanism by Which Rotavirus Impairs the Expression and Enzyme Activity of Brush Border-Associated Sucrase-Isomaltase in Differentiated Intestinal Caco-2 Cells," Cell. Microbiol. 6(8), 719-731.
28. Shahidi, M. et al. (2014) "Endocannabinoid Cb1 Antagonists Inhibit Hepatitis C Virus Production, Providing a Novel Class of Antiviral Host-Targeting Agents," J. Gen. Virol. 95(Pt 11), 2468-2479.
29. Gaunt, E. R. et al. (2013) "Inhibition of Rotavirus Replication by Downregulation of Fatty Acid Synthesis," J. Gen. Virol. 94(Pt 6), 1310-1317.
30. Berardi, V. et al. (2009) "Resveratrol Exhibits a Strong Cytotoxic Activity in Cultured Cells and Has an Antiviral Action against Polyomavirus: Potential Clinical Use," J. Exp. Clin. Cancer Res. 28, 96.
31. Palamara, A. T. et al. (2005) "Inhibition of Influenza a Virus Replication by Resveratrol," J. Infect. Dis. 191(10), 1719-1729.
32. Medina-Bolivar, L. F. *et al.* "Production of Stilbenes in Plant Hairy Root Cultures," United States Patent 7,666,677, Application 11/773,178, filed 7/3/2007. (issued 2/23/2010).
33. Mattion, N. M. et al. (1992) "Characterization of an Oligomerization Domain and RNA-Binding Properties on Rotavirus Nonstructural Protein Ns34," Virology 190(1), 68-83.
34. Freshney, R. I., (Ed.) (1994) Culture of Animal Cells. A Manual of Basic Technique, 3rd ed., Wiley-Liss, Inc, New York.
35. Arnold, M. et al. (2009) "Culturing, Storage, and Quantification of Rotaviruses," in Current Protocols in Microbiology, John Wiley & Sons, Inc.
36. Kitamoto, N. et al. (1991) "Comparative Growth of Different Rotavirus Strains in Differentiated Cells (Ma104, Hepg2, and Caco-2)," Virology 184(2), 729-737.
37. HUANG, H. et al. (2004) "Interaction(S) of Rotavirus Non-Structural Protein 4 (Nsp4) C-Terminal Peptides with Model Membranes," Biochem. J. 380(Pt 3), 723-733.
38. Swaggerty, C. L. et al. (2004) "Comparison of Sivmac239(352-382) and Sivsmmpbj41(360-390) Enterotoxic Synthetic Peptides," Virology 320(2), 243-257.

## Claims

1. A prenylated stilbenoid for use in a method for inhibiting a rotavirus infection in a subject, comprising administering to said subject exhibiting at least one symptom of a rotavirus infection a pharmaceutically effective amount of at least one prenylated stilbenoid, under conditions such that said at least one symptom of a rotavirus infection is reduced.

2. The prenylated stilbenoid for the use of claim 1, wherein said at least one prenylated stilbenoid comprises a combination of prenylated stilbenoids.

3. The prenylated stilbenoid for the use of claim 1, wherein said at least one prenylated stilbenoid is a purified stilbenoid.

4. The prenylated stilbenoid for the use of claim 1, wherein said at least one prenylated stilbenoid comprises an extract of hairy root cultures of the peanut plant.

5. The prenylated stilbenoid for the use of claim 1, wherein said at least one prenylated stilbenoid comprises an enriched fraction of a stilbenoid extract.

6. The prenylated stilbenoid for the use of claim 1, wherein said prenylated stilbenoid is selected from the group consisting of arachidin-1 and arachidin-3.

7. The prenylated stilbenoid for the use of claim 1, wherein said prenylated stilbenoid is purified from a hairy root culture.

8. The prenylated stilbenoid for the use of claim 5, wherein said extract enriched in prenylated stilbenoids is obtained from a hairy root culture.

9. The prenylated stilbenoid for the use of claim 1, wherein said prenylated stilbenoid is produced synthetically.

10. The prenylated stilbenoid for the use of claim 1, wherein said prenylated stilbenoid modulates at least one cannabinoid receptor

11. The prenylated stilbenoid for the use of claim 10, wherein said modulation of at least one cannabinoid receptor reduces virus replication.

12. The prenylated stilbenoid for the use of claim 1, wherein said prenylated stilbenoid modulates autophagy in virus infected cells.

13. The prenylated stilbenoid for the use of claim 12, wherein said modulation of autophagy reduces virus replication.

14. The prenylated stilbenoid for the use of claim 1, wherein said administering is oral.

## Patentansprüche

1. Prenyliertes Stilbenoid für die Verwendung in einem Verfahren zum Hemmen einer Rotavirusinfektion in einem Subjekt, umfassend Verabreichen einer pharmazeutisch wirksamen Menge von mindestens einem gereinigten prenylierten Stilbenoid an das Subjekt, das mindestens ein Symptom einer Rotavirusinfektion zeigt, unter solchen Bedingungen, dass mindestens eines der Symptome einer Rotavirusinfektion vermindert wird.

2. Prenyliertes Stilbenoid für die Verwendung nach Anspruch 1, wobei das mindestens eine prenylierte Stilbenoid eine Kombination von prenylierten Stilbenoiden umfasst.

3. Prenyliertes Stilbenoid für die Verwendung nach Anspruch 1, wobei das mindestens eine prenylierte Stilbenoid ein gereinigtes Stilbenoid ist.

4. Prenyliertes Stilbenoid für die Verwendung nach Anspruch 1, wobei das mindestens eine prenylierte Stilbenoid einen Extrakt aus Haarwurzelkulturen der Erdnusspflanze umfasst.

5. Prenyliertes Stilbenoid für die Verwendung nach Anspruch 1, wobei das mindestens eine prenylierte Stilbenoid eine angereicherte Fraktion eines Stilbenoid-Extrakts umfasst.

6. Prenyliertes Stilbenoid für die Verwendung nach Anspruch 1, wobei das prenylierte Stilbenoid ausgewählt ist aus der Gruppe bestehend aus Arachidin-1 und Arachidin-3.

7. Prenyliertes Stilbenoid für die Verwendung nach Anspruch 1, wobei das prenylierte Stilbenoid aus einer Haarwurzelkultur purifiziert ist.

8. Prenyliertes Stilbenoid für die Verwendung nach Anspruch 5, wobei der an prenylierten Stilbenoiden angereicherte Extrakt aus einer Haarwurzelkultur erhalten wird.

9. Prenyliertes Stilbenoid für die Verwendung nach Anspruch 1, wobei das prenylierte Stilbenoid synthetisch erzeugt wird.

10. Prenyliertes Stilbenoid für die Verwendung nach Anspruch 1, wobei das prenylierte Stilbenoid mindestens einen Cannabinoid-Rezeptor moduliert.

11. Prenyliertes Stilbenoid für die Verwendung nach Anspruch 10, wobei die Modulation mindestens eines Cannabinoid-Rezeptors Virusreplikation reduziert.

12. Prenyliertes Stilbenoid für die Verwendung nach Anspruch 1, wobei das prenylierte Stilbenoid Autophagie in virusinfizierten Zellen moduliert.

13. Prenyliertes Stilbenoid für die Verwendung nach Anspruch 12, wobei die Modulation der Autophagie Virusreplikation reduziert.

14. Prenyliertes Stilbenoid für die Verwendung nach Anspruch 1, wobei das Verabreichen oral erfolgt.

## Revendications

1. Stilbénoïde prénylé pour l'utilisation dans un procédé pour inhiber une infection à rotavirus chez un sujet, comprenant l'administration audit sujet présentant au moins un symptôme d'une infection à rotavirus d'une quantité pharmaceutiquement efficace d'au moins un stilbénoïde prénylé, dans des conditions telles que ledit au moins un symptôme d'une infection à rotavirus est réduit.

2. Stilbénoïde prénylé pour l'utilisation selon la revendication 1, dans lequel ledit au moins un stilbénoïde prénylé comprend une combinaison de stilbénoïdes prénylés.

3. Stilbénoïde prénylé pour l'utilisation selon la revendication 1, dans lequel ledit au moins un stilbénoïde prénylé est un stilbénoïde purifié.

4. Stilbénoïde prénylé pour l'utilisation selon la revendication 1, dans lequel ledit au moins un stilbénoïde prénylé comprend un extrait de cultures de racines chevelues de la plante d'arachide.

5. Stilbénoïde prénylé pour l'utilisation selon la revendication 1, dans lequel ledit au moins un stilbénoïde prénylé comprend une fraction enrichie d'un extrait de stilbénoïde.

6. Stilbénoïde prénylé pour l'utilisation selon la revendication 1, lequel stilbénoïde prénylé est choisi dans le groupe constitué par l'arachidine-1 et l'arachidine-3.

7. Stilbénoïde prénylé pour l'utilisation selon la revendication 1, lequel stilbénoïde prénylé est purifié à partir d'une culture de racines chevelues.

8. Stilbénoïde prénylé pour l'utilisation selon la revendication 5, dans lequel ledit extrait enrichi en stilbénoïdes prénylés est obtenu à partir d'une culture de racines chevelues.

9. Stilbénoïde prénylé pour l'utilisation selon la revendication 1, lequel stilbénoïde prénylé est produit synthétiquement.

10. Stilbénoïde prénylé pour l'utilisation selon la revendication 1, lequel stilbénoïde prénylé module au moins un récepteur cannabinoïde.

11. Stilbénoïde prénylé pour l'utilisation selon la revendication 10, dans lequel ladite modulation d'au moins un récepteur cannabinoïde réduit la réplication virale.

12. Stilbénoïde prénylé pour l'utilisation selon la revendication 1, lequel stilbénoïde prénylé module l'autophagie dans les cellules infectées par le virus.

13. Stilbénoïde prénylé pour l'utilisation selon la revendication 12, dans lequel ladite modulation d'autophagie réduit la réplication virale.

14. Stilbénoïde prénylé pour l'utilisation selon la revendication 1, dans lequel ladite administration est orale.
